# EUROPEAN PATENT APPLICATION

(11) **EP 4 559 526 A2**
(43) Date of publication of application: **28.05.2025**
(21) Application number: 25151598.7
(22) Date of filing: 19.07.2019
(51) Int. Cl.: A61P 35/00

(54) **COMPOSITION FOR REGULATING CANCER CELL DIVISION OR DIFFERENTIATION COMPRISING SETDB1 OR A SETDB1 INHIBITOR**

(30) Priority: 19.07.2018 KR 20180084090
(62) Divisional of application: 19187282.9
(71) Applicant: Korea Advanced Institute of Science and Technology, Daejeon 34141 (KR)
(72) Inventor: CHO, Kwang Hyun, Yuseong-gu, Daejeon 34141 (KR); LEE, Soo Beom, Yuseong-gu, Daejeon 34141 (KR)
(74) Representative: D Young & Co LLP

(57) **Abstract**

The present invention relates to a method for reverting cancer cells to normal cells using a specific molecular target. The present inventors have newly identified that SETDB1 according to the present disclosure is a key protein regulating cancer growth and differentiation ability, and that the SETCD1 inhibition may allow cancer cell malignancy to be markedly reduced and may promote differentiation of the cancer cell into the same state as normal cells. Thus, the SETDB1 not only provides a novel molecular target for the prevention or treatment of cancer, but also may be useful for the development of anti-cancer drugs.

## Description

### CROSS-REFERENCE TO RELATED APPLICATIONS

This application is based on and claims priority from Korean Patent Application No. 10-2018-0084090, filed on July 19^{th}, 2018, with the Korean Intellectual Property Office, the disclosure of which is incorporated herein in its entirety by reference.

### TECHNICAL FIELD

The present disclosure relates to a method for reverting cancer cells to normal cells using a specific molecular target. Specifically, the present disclosure relates to a composition for regulating cancer cell division or differentiation comprising SETDB 1 (SET domain, bifurcated 1) or a SETDB 1 inhibitor, or a composition for preventing or treating cancer comprising a SETDB 1 inhibitor.

### BACKGROUND

So far, cancer cell therapy strategies have been focused on inducing cancer cell death. However, anti-cancer agents that induce the death of cancer cells have encountered limitations due to acquisition of resistance of cancer cells against anti-cancer agents and inherent heterogeneity of cancer cells.

Several alternative therapies have been proposed to overcome these limitations. Among them, cancer treatment using cell differentiation is limited, but has been effectively applied to some blood cancers. Typically, differentiation induction therapy agents for acute promyelocytic leukemia has been developed and then achieved 80 to 90% cure rate of clinical outcomes and is currently used as a standard treatment for acute promyelocytic leukemia. These differentiation induction therapy methods are known to have fewer adverse effects as well as good therapeutic effects in comparison with conventional cancer therapy aiming at the death of cancer cells. This is because when cancer cells are differentiated, the rapid growth rate of cancer cells and metastasis thereof to other tissues disappear, and the cells do not undergo cell division as the final differentiated normal cells. Despite the above advantages, effective molecular targets for solid cancers have not yet been identified. Therefore, there is a considerable lack of new drug development.

### SUMMARY

The present inventors have sought to find a molecular target that differentiates cancer cells into normal cells. Thus, we have identified that the SETDB 1 gene, one of the histone-lysine N-methyltransferases, is abnormally overexpressed in some cancer cells with high differentiation ability and maintains high differentiation ability of cancer stem cells. Further, we identified that when SETDB 1 is suppressed, cancer cells are differentiated into a state similar to normal cells, thereby completing the present invention.

A purpose of the present disclosure is to provide a composition for regulating cancer cell division or differentiation, the composition comprising SETDB1 or a SETDB 1 inhibitor.

Another purpose of the present disclosure is to provide a composition for the prevention or treatment of cancer, including a SETDB 1 inhibitor.

Another purpose of the present disclosure is to provide a method for inducing differentiation of cancer stem-like cells into normal cells or normal-like cells using SETDB 1 or a SETDB 1 inhibitor and a method for screening an agent for preventing or treating cancer using SETDB 1 or a SETDB 1 inhibitor.

Another purpose of the present disclosure is to provide the SETDB 1 inhibitor for use in the treatment of cancer.

An exemplary embodiment of the present disclosure provides a composition for regulating cancer cell division or differentiation, the composition comprising SETDB 1 or a SETDB 1 inhibitor.

Further, another exemplary embodiment of the present disclosure provides a pharmaceutical composition for the prevention or treatment of cancer, the composition comprising a SETDB 1 inhibitor.

Further, another exemplary embodiment of the present disclosure provides an *in vitro* method of inducing differentiation of cancer stem-like cells into normal cells or normal-like cells, the method comprising applying SETDB1 or a SETDB1 inhibitor to the cancer stem-like cells.

Yet another exemplary embodiment of the present disclosure provides a method for screening an agent for preventing or treating cancer, the method comprising (a) applying a candidate substance to an isolated cancer cell expressing SETDB1; (b) measuring a SETDB 1 expression or activity level in the isolated cancer cell to which the candidate substance is applied; and (c) selecting the candidate substance as an agent for preventing or treating cancer when the SETDB 1 expression or activity level measured in the step (b) is lower than a SETDB 1 expression or activity level in an isolated cancer cell to which the candidate substance is not applied.

Further, yet another exemplary embodiment of the present disclosure provides a SETDB 1 inhibitor for use in the treatment of cancer.

According to the exemplary embodiments of the present disclosure, the present inventors have newly identified that the SETDB1 is a key protein regulating cancer growth and differentiation ability and that when suppressing the SETDB1, cancer cell malignancy is markedly reduced and differentiation of the cancer cells into normal-like cells is promoted. Thus, the SETDB1 not only provides a novel molecular target for the prevention or treatment of cancer, but also is useful for the development of anti-cancer drugs.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF THE DRAWINGS

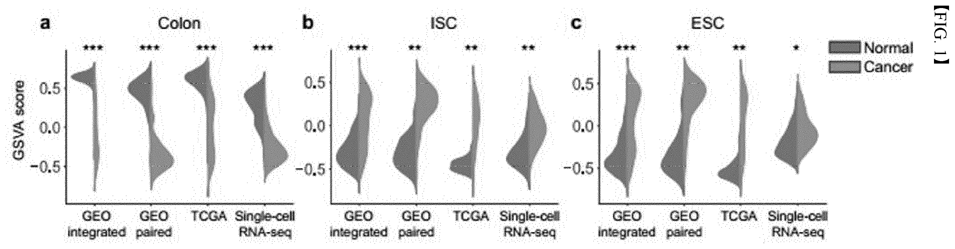

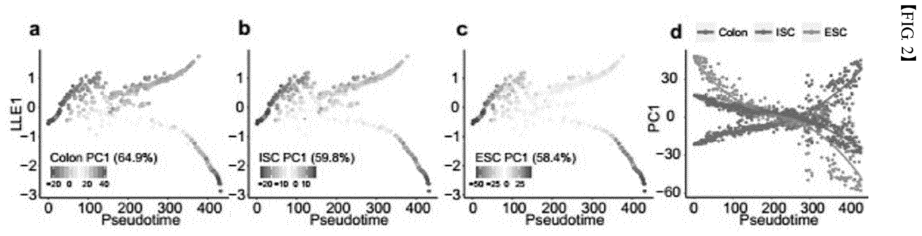

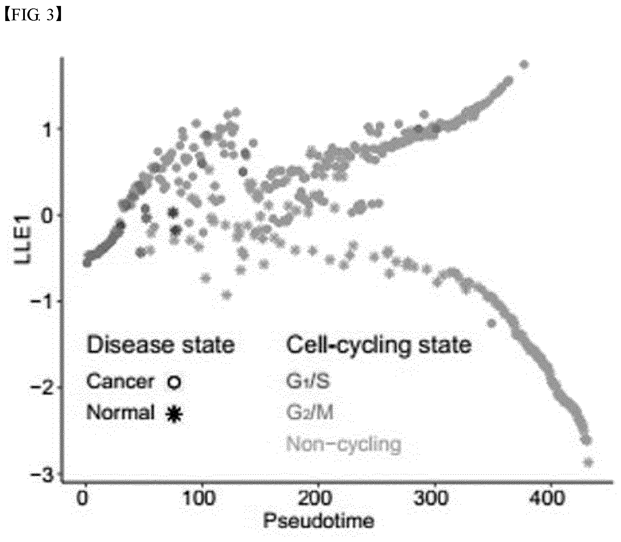

FIGS. 1 to 3 show the results of comparative analysis of normal colon tissue samples and colorectal cancer tissue samples as extracted from the GEO database and the TCGA database. Specifically, FIGS. 1A to 1C show violin plots showing gene set enrichment analysis (GSEA) score of each tissue-specific gene set in normal tissues and cancer tissues across the datasets. FIGS. 2A to 2C show inferred pseudotime (x-axis) and first dimension (y-axis) of linear local embedding (LLE), which is a nonlinear dimensionality reduction implemented by SLICER algorithm. Principal component 1 (PC1) of each gene set was marked with colored points. The proportion of variance explained by PC1 is shown at the bottom. FIG. 2D shows smoothened curve of PC1 of each tissue-specific gene set along pseudotime. FIG. 3 shows scatter plot showing cycling cells among total cell population.

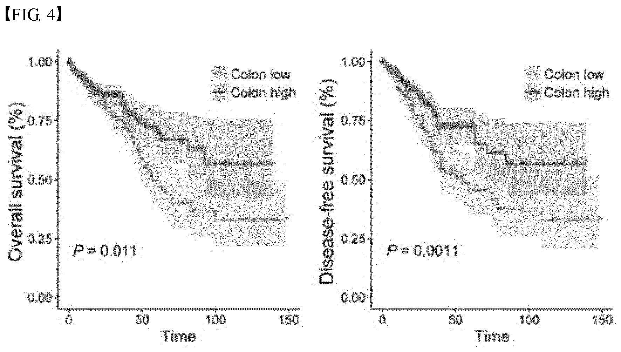

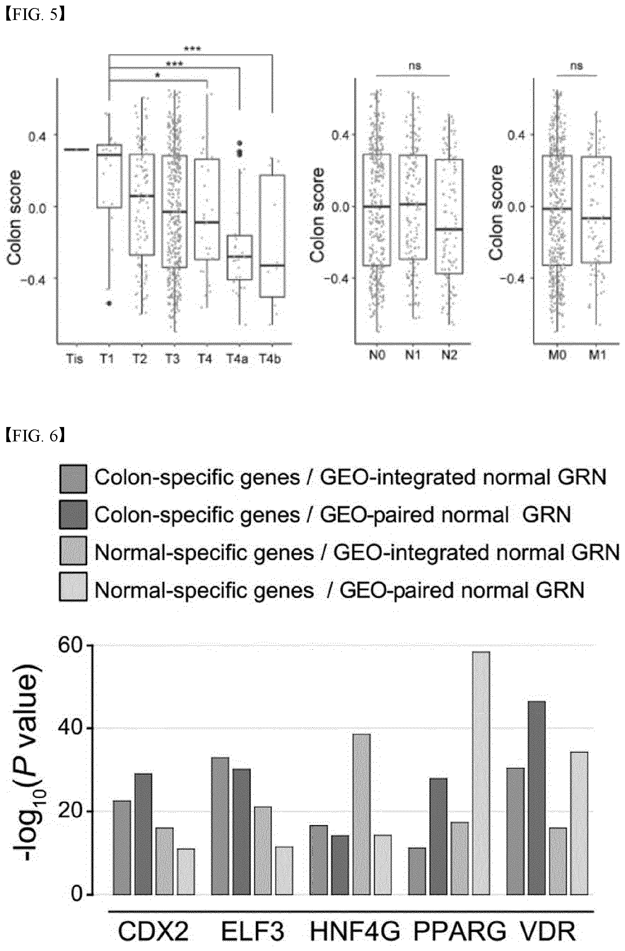

FIGS. 4 and 5 show the results of identifying the clinical relevance of specific genetic expression patterns of normal colon cells. FIG. 4 shows graphs of Kaplan-Meier analysis of overall survival and disease-free survival. FIG. 5 shows box plots showing the GSVA score of colon-specific gene set. The extent of the tumor (left), the extent of spread to the lymph nodes (middle), and the presence of distant metastasis (right). (*P < 0.05, **P < 0.01, ***P < 0.001)

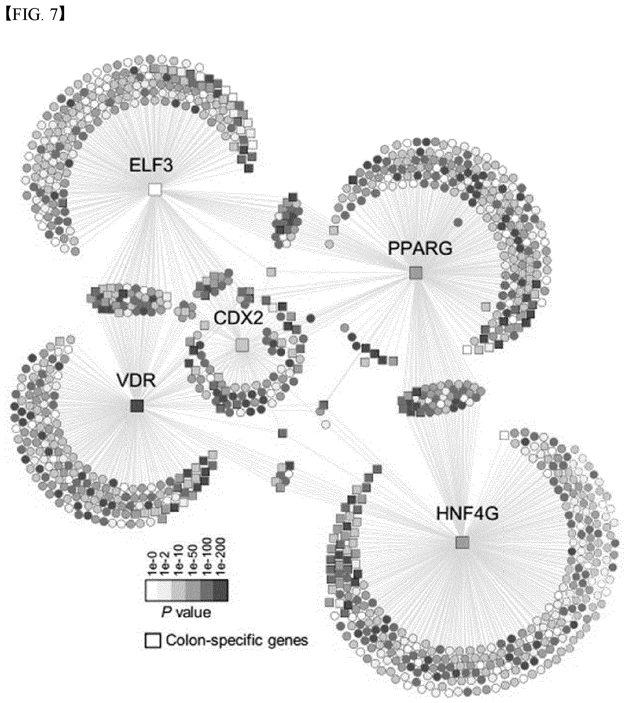

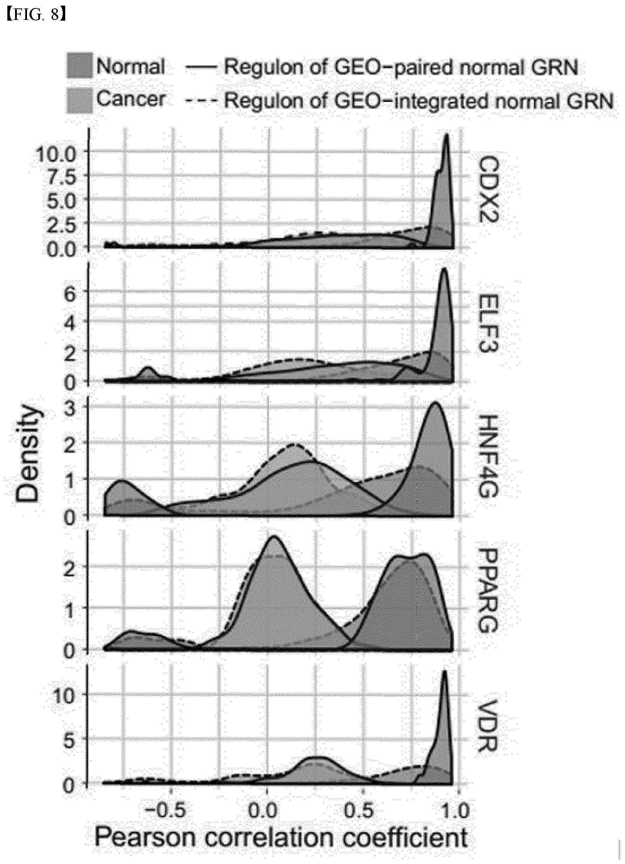

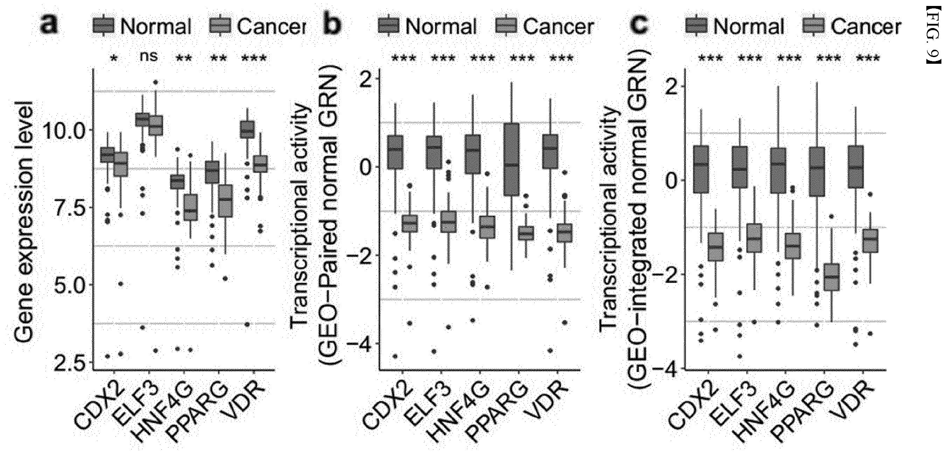

FIG. 6 to FIG. 9 show the results of identifying the core transcription factors of normal colon epithelial cells. Specifically, FIG. 6 shows the core transcription factor according to the MARINA analysis. FIG. 7 shows visualization of the core TFs and their target genes based on GEO-integrated normal GRN (Genetic control network inferred from GEO-paired normal data sets). The square nodes represent colon-specific genes. FIG. 8 shows histograms showing Pearson correlation coefficient between each core TF and its regulon. The regulons from GEO-paired normal GRN (solid line), and the regulons from GEO-integrated normal GRN (dashed line) are shown. FIG. 9A shows the results of comparison of gene expression level of core TFs in GEO-paired dataset. FIGS. 9B and 9C show transcriptional activity of the core TFs inferred by VIPER algorithm in GEO-paired normal GRN (left) and GEO-integrated normal GRN (right). (*P < 5e-2, **P < 1e-10, ***P < 1e-20).

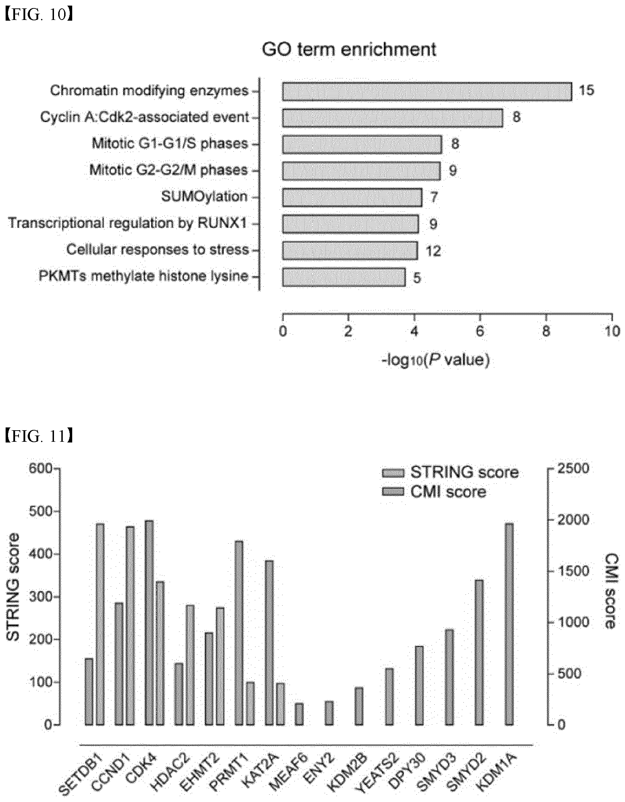

FIG. 10 and FIG. 11 show the results of screening potential regulators or causative genes (chromatin regulator) that inhibit the activity of core transcription factors that regulate the specific gene expression pattern of normal colon cells. FIG. 10 shows the result of GO term enrichment analysis. FIG. 11 shows the results of the STRING database and CMI score analysis for 15 chromatin regulators selected from the GO term enrichment analysis.

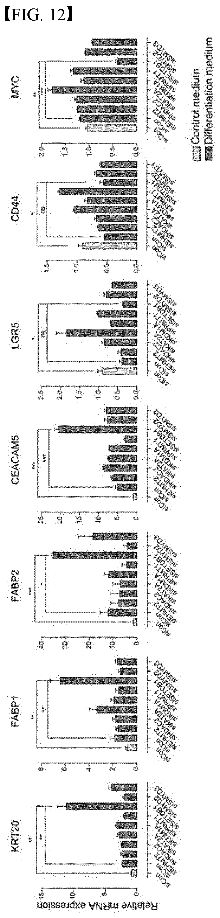

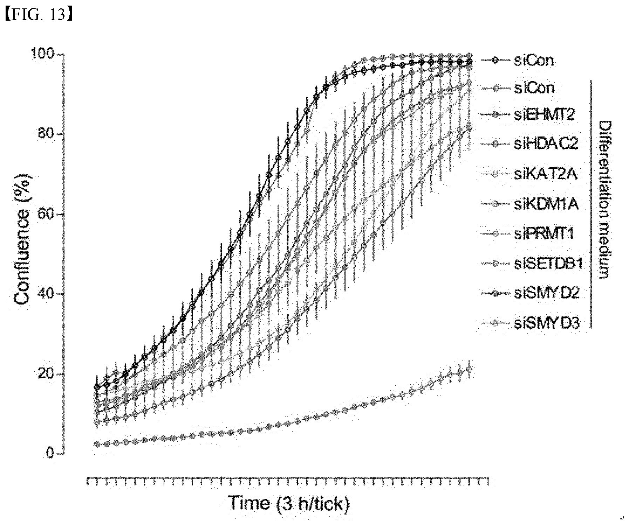

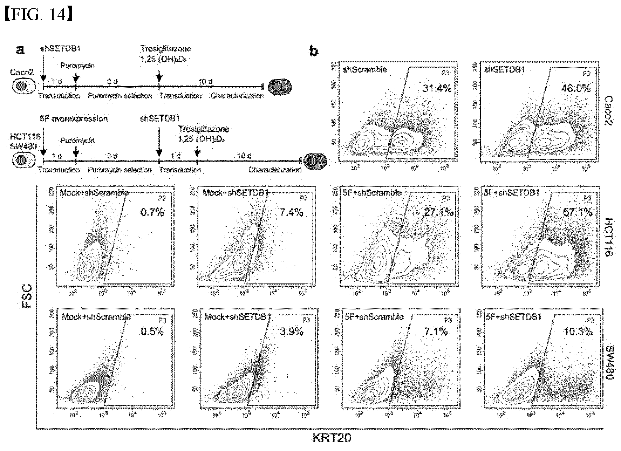

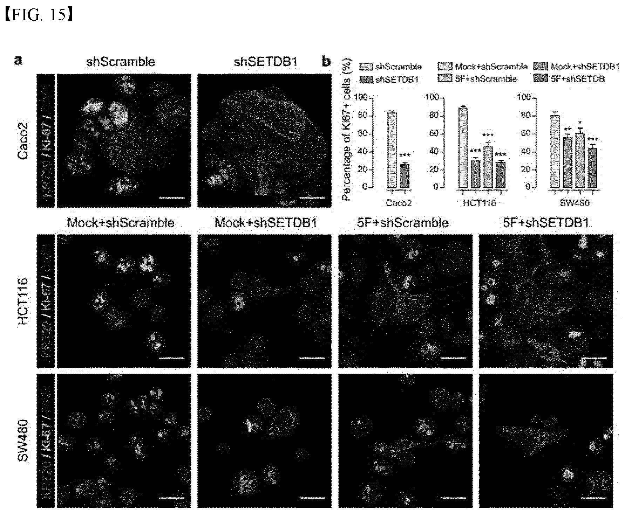

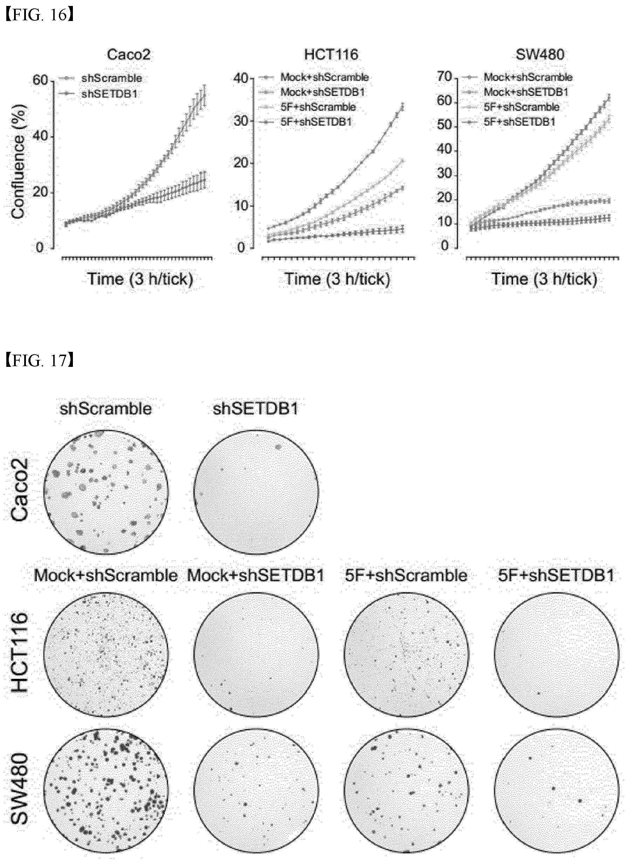

FIGS. 12 to 17 show the results of screening for the gene (chromatin regulator) responsible for the inhibition of activity of the core transcription factor. FIG. 12 shows the results of qRT-PCR analysis of the expression level of differentiation markers and stem cell-associated genes after transfection of 8 chromatin regulators with siRNA into Caco2 cells. FIG. 13 shows percent growth (%) over time in Caco2 after transfection with the indicated siRNAs. FIG 14A shows a protocol of the experiments with retrovirus-mediated transduction. FIG. 14B shows flow cytometry data showing KRT20 abundance (x-axis) and forward-scattered light (FSC) (y-axis). FIG. 15A shows the results of immunofluorescence of KRT20 (red) and Ki-67 (green) with DAPI nuclear counterstain (blue) (Scale bar = 50 µm). FIG. 15B shows the results of quantification of Ki-67-positive cells in low magnification confocal immunofluorescence images for each group. (mean ±s.e.m.). FIG. 16 shows percent growth (%) of three cell lines Caco2, HCT116 and SW480 after SETDB1 knockdown. FIG. 17 shows the results of colony forming assay over 14 days. (*P < 0.05, **P < 0.01, ***P < 0.001)

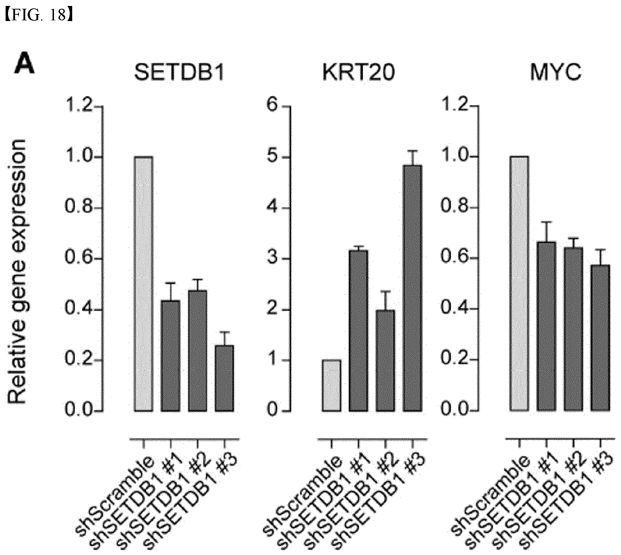

FIG. 18 shows the results of qRT-PCR analysis of the expression levels of differentiation markers and stem cell-associated genes when various shRNAs prepared for SETDB1 were applied to colon epithelial cells.

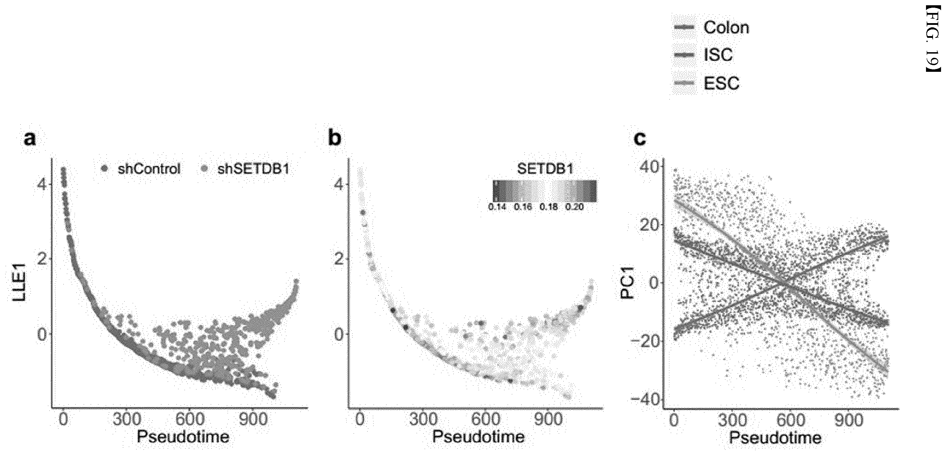

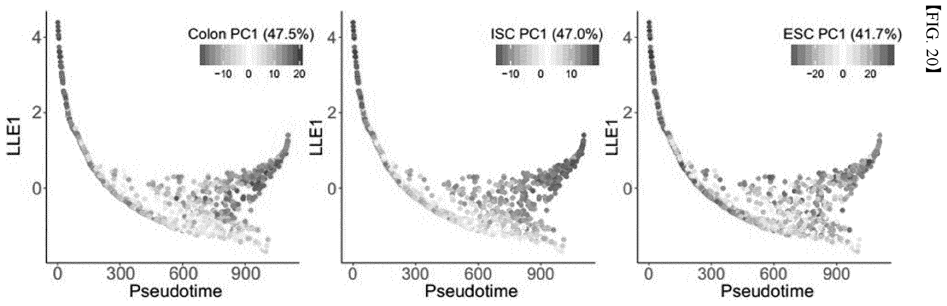

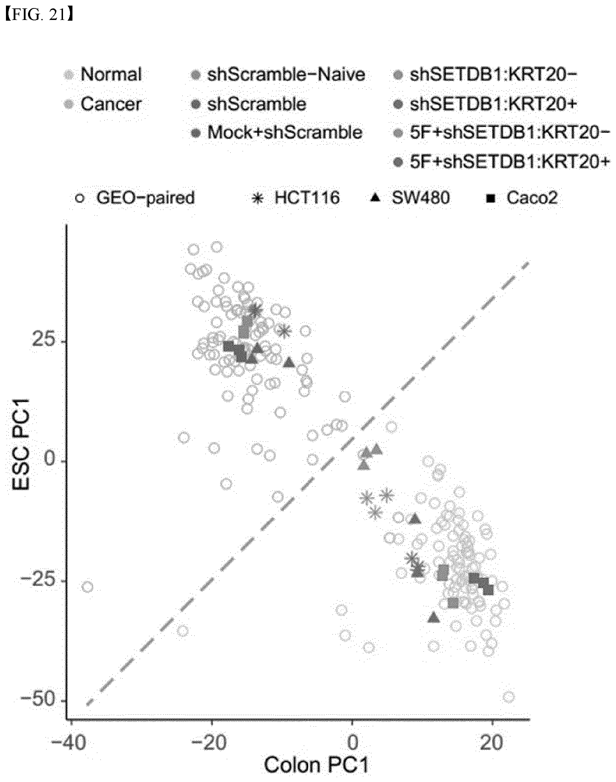

FIGS. 19 to 21 show the results of identifying the expression patterns of genes in differentiated normal-like cells. Specifically, FIG. 19A shows scatter plot showing the first dimension of LLE constructed by SLICER along pseudotime; FIG. 19B shows imputed gene expression level of SETDB1; FIG. 19C shows scatter plot of PC1 of each state-specific gene set. FIG. 20 shows scatter plots of state-specific gene set. In FIG. 21, bulk RNA-sequencing data of colon-specific genes (x-axis) and PC1 of ESC-specific genes (y-axis) are shown. GEO-paired dataset and *in vitro* samples are shown with different shapes.

### DETAILED DESCRIPTION

In the following detailed description, reference is made to the accompanying drawing, which forms a part hereof. The illustrative embodiments described in the detailed description, drawing, and claims are not meant to be limiting. Other embodiments may be utilized, and other changes may be made, without departing from the spirit or scope of the subject matter presented here.

The present disclosure is described in detail below.

The present disclosure provides a composition for regulating cancer cell division or differentiation, the composition comprising SETDB 1 or a SETDB1 inhibitor.

The SETDB1 (SET domain, bifurcated 1) according to the present disclosure is characterized by promoting the division of cancer cells and inhibiting differentiation thereof. The inhibitor of SETDB1 according to the present disclosure is characterized by inhibiting the division of cancer cells and promoting differentiation thereof.

The normal somatic cells may be converted to another developmental state by a transcription factor (TF). Cancer cells have characteristics that they are less differentiated than adjacent normal cells in tissues. Thus, the present inventors tried to confirm whether cancer cells could be induced to normal state by further differentiation of the cancer cells as normal cells.

First, in an embodiment of the present disclosure, to identify transcription factors that convert stem cell-like colorectal cancer cells (or colorectal cancer stem-like cells) into differentiated normal-like cells, we analyzed large-scale public genomic data, and thus found out that the difference between normal colon cells and cancer cells was related to in the amount of colon-specific gene expression. Thus, we identified that the colorectal cancer (CRC) cells have different developmental stages from embryonic and intestinal stem cell-like cells with high differentiation ability to differentiated normal-like cells with gene expression patterns similar to gene expression patterns of normal cells. On the basis of this finding, we have proposed a treatment method to eliminate cancer malignancy by converting the cancer stem cell known to play a central role in cancer growth to normal cells. For this purpose, genomic data of normal colon tissues were collected and synthesized to construct a gene regulatory network (GRN) of normal colon mucosa cells. Then, we confirmed that CDX2, ELF3, HNF4G, PPARG and VDR were the core transcription factors regulating cell differentiation. Further, we confirmed that cancer cells had a significant decrease in the protein activity thereof.

Furthermore, to search for a gene that inhibits the protein activity of core transcription factors, the causative genes were searched for from the genomic data of colon cancer patient tissues. Thus, we executed screening experiments to identify that SETDB1 was the causative gene. The SETDB1 is one of the histone-lysine N-methyltransferases. It is known that the SETDB1 has the function of inhibiting the expression of a specific gene by attaching a methyl group to the histone protein. Then, the present inventors have identified for the first time that the above gene SETDB1 is abnormally over-expressed in some colorectal cancer cells with high differentiation ability to keep differentiation ability of cancer stem cells (or cancer stem-like cells) high. Finally, using single-cell RNA sequencing (RNA-seq) and bulk RNA sequencing analysis, we confirmed that when the SETDB 1 was inhibited, the colorectal cancer cells were differentiated into a state (post-mitotic state) similar to normal colon cells and their growth rate was greatly inhibited.

As used herein, the term "cancer stem cell" refers to a generic term meaning cancer cells that have regenerating or differentiating ability as the unique ability to stem cells. Recently, it has been discovered that cells inducing the cancer are separately present in cancer cells, and the cells inducing the cancer have the characteristics of normal stem cells and may be able to cause the cancer again. Thus, these cells inducing the cancer have the characteristics of the stem cells and thus are called "cancer stem cells".

As used herein, the term "cancer stem cell" may be used interchangeably with "cancer stem-like cell". Any cell may be referred to as the cancer stem-like cell as long as it may differentiate into general cancer cells.

According to the present disclosure, the SETDB1 inhibitor may be an expression inhibitor of the SETDB 1 gene or an activity inhibitor of the SETDB 1 protein. The expression inhibitor of the SETDB1 gene may be at least one selected from the group consisting of an antisense oligonucleotide, siRNA (small interfering RNA), shRNA (small hairpin RNA) and miRNA (microRNA) that bind complementarily to the mRNA of a gene that promotes expression of SETDB 1 or SETDB1 gene. However, the present disclosure is not limited thereto. Further, the activity inhibitor of the SETDB1 protein may be at least one selected from the group consisting of an antibody, an antigen-binding fragment thereof, a compound, a peptide, a peptide mimetic, and an aptamer which specifically bind to the SETDB1 protein. However, the present disclosure is not limited thereto.

In one example, the compound is not limited as long as it inhibits the activity of SETDB 1 protein and may be included in the present disclosure. The compound may be a naturally occurring substance or a synthesized substance and may be preferably a low molecular weight compound.

As used herein, the term "antisense oligonucleotide" refers to DNA or RNA or derivatives thereof containing a nucleic acid sequence complementary to a sequence of a specific mRNA. The antisense oligonucleotide binds to a complementary sequence in mRNA and thus acts to inhibit the translation of the mRNA into protein. The antisense sequence means a DNA or RNA sequence complementary to the mRNA of the gene and capable of binding to the mRNA. The antisense sequence may suppress essential activity for translation, translocation into the cytoplasm, maturation, or any other overall biological functions of the mRNA.

Further, the antisense nucleic acid may be modified at one or more bases, sugar or backbone positions thereof to enhance efficacy thereof. The nucleic acid backbone may be modified into phosphorothioate, phosphotriester, methylphosphonate, short chain alkyl, cycloalkyl, short chain heteroatomic, heterocyclic inter-sugars bond, and the like. Further, the antisense nucleic acid may contain one or more substituted sugar moieties. The antisense nucleic acid may contain a modified base. The modified bases may include hypoxanthane, 6-methyladenine, 5-methylpyrimidine (especially 5-methylcytosine), 5-hydroxymethyl cytosine (HMC), glycosyl HMC, zentobiosyl HMC, 2-amino adenine, 2-thiouracil, 2-thiothymine, 5-bromouracil, 5-hydroxymethyluracil, 8-azaguanine, 7-deazaguanine, N6(6-aminohexyl)adenine, 2,6-diaminopurine, etc. Further, the antisense nucleic acid can be chemically combined with one or more moieties or conjugates that enhance the activity and cellular adsorption of the antisense nucleic acid. The moieties may include fat-soluble moieties such as cholesterol moiety, cholesteryl moiety, cholic acid, thioether, thiocholesterol, fatty chains, phospholipids, polyamines, polyethylene glycol chains, adamantane acetic acid, palmityl moiety, octadecylamine, hexylaminocarbonyl-oxycholesterol moiety, and the like, but are not limited thereto. The antisense oligonucleotides may be synthesized *in vitro* in a conventional manner and administered *in vivo.* Alternatively, the antisense oligonucleotides may be synthesized *in vivo.*

As used herein, the term "siRNA" refers to a nucleic acid molecule capable of mediating RNA interference or gene silencing. Because siRNAs can inhibit the expression of target genes, they are provided for efficient gene knockdown or gene therapy methods.

In the siRNA molecule according to the present disclosure, the sense strand and the antisense strand are located to be opposite to each other to form a double strand structure. In this connection, the sense strand may refer to a sequence corresponding to the mRNA sequence of the target gene, the SETDB1 gene, according to one embodiment according to the present disclosure. The antisense strand may refer to a complementary sequence to the mRNA sequence. The siRNA molecules according to the present disclosure may have a single chain structure with self-complementary sense and antisense strands. Furthermore, siRNAs are not limited to the complete pairing of double-stranded RNA portions in which RNAs pair with each other. Rather, the unpaired portion due to a mismatch (the corresponding base is not complementary thereto), a bulge (no base corresponding to one of the chains), and the like may be contained in the siRNA. Further, as long as the siRNA terminal structure can inhibit the expression of the target gene by the RNAi effect, the siRNA terminal structure may be both a blunt terminal and a cohesive terminal. The cohesive terminal structure may be both a 3'-terminal protruding structure and a 5'-terminal protruding structure.

The method for preparing siRNA includes a method of directly synthesizing siRNA in a test tube, and then introducing the siRNA into a cell via a transformation process, and a method of transforming or infecting, into the cells, a siRNA expression vector or a PCR-derived siRNA expression cassette as produced so that a siRNA is expressed in a cell.

The siRNA may be, for example, a siRNA represented by any one of SEQ ID NOs. 4 to 19 or may be preferably a siRNA targeting SETDB 1 as represented by SEQ ID NO. 14 or SEQ ID NO. 15. However, the present disclosure is not limited thereto.

As used herein, "shRNA" refers to small hairpin RNA or short hairpin RNA and is used to silence genes via RNA interference. The shRNA may be generally introduced into the target cell using a vector. This shRNA hairpin structure is cleaved by other substances in the cell to become siRNA.

The shRNA may be, for example, but is not limited to, a shRNA represented by any of SEQ ID NOs: 1 to 3.

Further, according to the present disclosure, the SETDB 1 inhibitor inhibits the transcriptional activity of at least one protein selected from the group consisting of CDX2 (caudal type homeobox 2), ELF3(E74 like ETS transcription factor 3), HNF4G(hepatocyte nuclear factor 4 gamma), PPARG(peroxisome proliferator-activated receptor gamma), and VDR(vitamin D receptor).

According to the present disclosure, the cancer cell may be a cancer stem-like cell.

Further, the present disclosure provides a pharmaceutical composition for the prevention or treatment of cancer, the composition comprising a SETDB 1 inhibitor.

According to the present disclosure, the cancer may be solid cancer, and may preferably be at least one selected from the group consisting of colon cancer, rectal cancer, liver cancer, stomach cancer, lung cancer, breast cancer and pancreatic cancer. More preferably, the cancer may be a colorectal cancer or rectal cancer, most preferably a colon cancer. The cancer may not be limited to a specific cancer as long as the cancer development such as tumor differentiation or proliferation is dependent on cancer stem cell as described herein.

According to the present disclosure, the SETDB1 inhibitor may be an expression inhibitor of the SETDB 1 gene or an activity inhibitor of the SETDB 1 protein. Preferably, the SETDB 1 inhibitor may be an expression inhibitor of the SETDB 1 gene. The expression inhibitor of the SETDB 1 gene may be at least one selected from the group consisting of an antisense oligonucleotide, siRNA (small interfering RNA), shRNA (small hairpin RNA) and miRNA (microRNA) that bind complementarily to the mRNA of a gene that promotes expression of SETDB 1 or SETDB 1 gene. However, the present disclosure is not limited thereto.

In one embodiment according to the present disclosure, the division of cancer cells is inhibited, and its differentiation is promoted according to the shRNA application using the shRNAs indicated as SEQ ID NOs. 1 to 3 as the shRNA, thereby to induce the cancer cells into normal cells. Further, in one embodiment according to the present disclosure, when the siRNA indicated as SEQ ID NO. 14 or SEQ ID NO. 15 as the siRNA may be transfected into Caco2 cells as colon-derived colorectal adenocarcinoma cells and the epithelial cells, the high knockdown efficiency of the SETDB1 gene is confirmed.

According to the present disclosure, the SETDB1 is abnormally overexpressed in some cancers, preferably colorectal cancer cells, with a high differentiation ability, thus maintaining a high differentiation ability of cancer stem cells (or cancer stem-like cells). Thus, using the SETDB 1 inhibitor, cancer cells may differentiate into post-mitotic states similar to normal cells, and thus, their growth rate is greatly inhibited. Therefore, the pharmaceutical composition for the prevention or treatment of cancer, the composition comprising a SETDB 1 inhibitor, according to the present disclosure may be used for differentiation treatment of cancer cells or cancer stem-like cells. Further, the SETDB 1 inhibitor according to the present disclosure may be used as a combination drug with an anti-cancer agent or a radiotherapeutic agent. Thus, drug resistance and recurrence of the cancer can be suppressed and thus the therapeutic effect can be enhanced.

The pharmaceutical composition for preventing or treating cancer according to the present disclosure can additionally be administered in combination with other anti-cancer drugs, and thus can be used to treat cancer stem cells as well as general cancer cells. The pharmaceutical composition can also be used as a pharmaceutical composition for inhibiting recurrence or metastasis of cancer.

According to the present disclosure, the pharmaceutical composition may be in the form of capsules, tablets, granules, injections, ointments, powders or beverages. The pharmaceutical composition may be characterized by being directed to humans. The pharmaceutical composition may be formulated in the form of an oral preparation, such as powders, granules, capsules, tablets, aqueous suspensions, an external preparation, a suppository, and a sterile injection solution according to a conventional method. However, the present disclosure may not be limited thereto.

The pharmaceutical composition according to the present disclosure may contain a pharmaceutically acceptable carrier. The pharmaceutically acceptable carrier may include a binder, a lubricant, a disintegrant, an excipient, a solubilizing agent, a dispersing agent, a stabilizer, a suspending agent, a pigment, a fragrance, etc. In the injectable preparation, the carrier may include a buffer, a preservative, an anhydrous agent, a solubilizer, an isotonic agent, a stabilizer, etc. In the topical administration, the carrier may include a base, an excipient, a lubricant, a preservative, etc. The formulations of the pharmaceutical composition according to the present disclosure may be prepared in various manners by mixing the composition with the pharmaceutically acceptable carrier as described above. For example, when orally administered, the composition may be prepared in the form of tablets, troches, capsules, elixirs, suspensions, syrups, wafers, etc. In the injections, the composition may be manufactured in unit dosage ampoules or in multiple-dose formulations. The composition may be produced in the forms of other solutions, suspensions, tablets, capsules, sustained-release preparations and the like.

In one example, examples of carriers, excipients and diluents suitable for formulation include lactose, dextrose, sucrose, sorbitol, mannitol, xylitol, erythritol, maltoditol, starch, acacia rubber, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate or mineral oil, etc. Examples of carriers, excipients and diluents suitable for formulation may further include a filler, an anticoagulant, a lubricant, a wetting agent, a flavoring agent, an emulsifier, an antiseptic, and the like.

The route of administration of the pharmaceutical composition according to the present disclosure may be selected from the group consisting of oral, intravenous, intramuscular, intraarterial, intramedullary, intrathecal, intracardiac, transdermal, subcutaneous, intraperitoneal, intranasal, intestinal, topical, sublingual, or rectal route. The present invention is not limited to this route. The "parenteral" administration includes subcutaneous, intradermal, intravenous, intramuscular, intraarticular, intrasynovial, intrasternal, intrathecal, intralesional and intracranial injection or infusion techniques. The pharmaceutical composition according to the present disclosure may be administered in the form of suppositories for rectal administration.

The dosage of the pharmaceutical composition according to the present disclosure may vary widely depending on various factors including the activity of the specific active ingredient used, age, body weight, general health, sex, diet, time of administration, route of administration, discharging rate, drug formulation and severity of certain diseases to be prevented or treated. The dosage of the pharmaceutical composition will vary depending on the condition of the patient, the weight, the severity of the disease, the type of drug, the route of administration and the period of time, but can be suitably selected by those skilled in the art. Preferably, all of the above factors are taken into account so that an amount capable of achieving a maximal effect in a minimal amount without side effects can be administered. More preferably, the dosage may be in a range of from 0.01 µg to 1,000 mg/kg of body weight/day as an effective dose, more preferably, from 0.1 µg to 100 mg/kg of body weight/day, which may be repeated several times a day. The dosage does not in any way limit the scope according to the present disclosure.

Further, the present disclosure provides anti-cancer adjuvants comprising the SETDB1 inhibitor.

As used herein, the term "anti-cancer adjuvant" means an agent capable of improving or increasing the anti-cancer effect of an anti-cancer agent. For example, when the anti-cancer adjuvant is used alone, the anti-cancer adjuvant does not exhibit anti-cancer activity. However, when the anti-cancer adjuvant is used in combination with an anti-cancer agent, the anti-cancer adjuvant may improve or enhance the anti-cancer effect of the anti-cancer agent.

In another example, an agent exhibiting a concentration-dependent anti-cancer activity is used in combination with an anti-cancer agent at a level that the agent exhibiting a concentration-dependent anti-cancer activity does not exhibit anti-cancer activity when the agent exhibiting a concentration-dependent anti-cancer activity is used alone. Thus, the agent exhibiting a concentration-dependent anti-cancer activity may be used as an anti-cancer adjuvant which can improve or enhance the anti-cancer effect of the anti-cancer agent. In this case, the agent exhibiting a concentration-dependent anti-cancer activity may be used as an anti-cancer agent or anti-cancer adjuvant depending on the administration concentration. As such, the agent exhibiting a concentration-dependent anti-cancer activity may be used as an anti-cancer adjuvant at a dose range that the agent exhibiting a concentration-dependent anti-cancer activity does not exhibit anti-cancer activity when only the agent exhibiting a concentration-dependent anti-cancer activity is used.

The anti-cancer adjuvant according to the present disclosure may be administered in combination with an anti-cancer agent or anti-cancer adjuvant to enhance the anti-cancer effect of the anti-cancer agent. In an example, the anti-cancer adjuvant according to the present disclosure may be co-administered with known anti-cancer drugs such as doxorubicin, etoposide, daunorubicin, and mitoxantrone.

The route of administration of the anti-cancer adjuvant may include any route as long as the adjuvant can reach the target tissue via the route. The anti-cancer adjuvant according to the present disclosure may be administered by intraperitoneal, intravenous, intramuscular or subcutaneous administration as desired. The anti-cancer adjuvant may be administered intradermally, orally, nasally, pulmonary, rectally. The present invention is not limited to such an administration route. Further, the anti-cancer adjuvant may be administered by any device capable of transferring the activity material to the target cell.

Further, the present disclosure provides a health functional food for cancer prevention or improvement, the food comprising a SETDB 1 inhibitor.

The health functional foods according to the present disclosure can be manufactured and processed in the form of tablets, capsules, powders, granules, liquids, and pills, etc. for prevention and improvement of cancer.

As used herein, the term "health functional food" refers to foods manufactured and processed using raw materials or ingredients with useful functions for human body in accordance with the Law No. 6727 on Health Functional Foods. The health functional food means the food as ingested for the purpose of regulating nutrients related to the structure and function of the human body or obtaining a beneficial effect for health uses such as physiological action.

The health functional foods according to the present disclosure may contain conventional food additives. The qualification of the food additives may comply with the provisions of the Food and Drug Administration Regulations as approved by the FDA and with General Regulations for Food Additives and General Testing Methods as approved by the FDA unless otherwise defined.

Commercially available items listed in the food additive code include, for example, chemical compounds such as ketones, glycine, calcium citrate, nicotinic acid, and cinnamic acid. The items included in the food additive code include natural additives such as persimmon pigment, licorice extract, crystalline cellulose, high pigment, and guar gum, and mixed preparations such as a sodium L-glutamate preparation, a noodle-added alkaline agent, a preservative agent, and a tar coloring agent.

For example, a health functional food in the form of a tablet may be produced as follows. A mixture of the SETDB 1 inhibitor as the active ingredient according to the present disclosure with excipients, binders, disintegrants and other additives is produced. The mixture is granulated by a conventional method. A lubricant may be added to the granulated mixture which may be subjected to compression molding. Alternatively, the mixture may be directly compression molded. Further, the tablet-type health functional foods may contain bitters or the like as needed.

A hard capsule in a capsule-type health functional food is prepared as follows. The mixture of the inhibitor of SETDB 1, an active ingredient according to the present disclosure and additives such as excipients may be produced. Then, the ordinary hard capsule is filled with the mixture. The soft capsule is prepared as follows. The inhibitor of SETDB 1, an active ingredient, is mixed with additives such as excipients to prepare a mixture. The mixture is filled into a capsule base such as gelatin. The soft capsule may contain a plasticizer such as glycerin or sorbitol, a coloring agent, a preservative and the like, if necessary.

A pill type health functional food is prepared as follows. The active ingredient SETDB 1 inhibitor according to the present disclosure may be mixed with an excipients, binders, disintegrant, etc. The mixture is molded by a conventionally known method. If necessary, the pill may be coated with white sugar or other coating materials. Alternatively, the surface of the pill may be coated with a material such as starch or talc.

The granular health functional foods may be prepared by granulating a mixture of an inhibitor of SETDB1, as an active ingredient according to the present disclosure, and excipients, binders, disintegrants, etc., using a known method. If necessary, a flavoring agent, a bitter, and the like may be contained in the granular health functional foods.

The health functional foods may include beverage, meat, chocolate, food, confectionery, pizza, ramen, other noodles, gums, candy, ice cream, alcoholic beverages, vitamin complexes, health supplement foods, and the like.

Further, the present disclosure provides an *in vitro* method for inducing differentiation of cancer stem-like cells into normal cells or normal-like cells, the method comprising applying SETDB 1 or a SETDB 1 inhibitor to the cancer stem-like cells.

Yet another exemplary embodiment of the present disclosure provides a method for screening an agent for preventing or treating cancer, the method comprising (a) applying a candidate substance to an isolated cancer cell expressing SETDB1; (b) measuring a SETDB 1 expression or activity level in the isolated cancer cell to which the candidate substance is applied; and (c) selecting the candidate substance as an agent for preventing or treating cancer when the SETDB 1 expression or activity level measured in the step (b) is lower than a SETDB 1 expression or activity level in an isolated cancer cell to which the candidate substance is not applied.

According to the screening method according to the present disclosure, a candidate substance to be analyzed may first contact a cancer cell containing the gene or protein. The candidate substances are unknown substances used in the screening to check whether they affect the expression level of the gene, the amount of protein or the activity of the protein. The sample may include, but is not limited to, a chemical, antisense oligonucleotide, antisense-RNA, siRNA, shRNA, miRNA, an antibody specific to the protein or natural extract.

Then, the amount of expression of the gene, the amount of protein, or the activity of protein can be measured in a cell to which the candidate substances are applied. When the measurement results show that the expression level of the gene, the amount of protein or the activity of the protein is decreased, it may be determined that the candidate substances can be used to treat or prevent cancer.

According to the present disclosure, the method of measuring the expression level of a gene or the amount of a protein can be performed using known techniques including known processes for separating mRNA or protein from a biological sample. As used herein, the term "biological sample" refers to a specimen taken from a living organism whose expression level or protein level of the gene according to the degree of cancer development or progression differs from those of the normal control group. The sample may include, but is not limited to, tissue, cells, blood, serum, plasma, saliva, urine, and the like.

The measurement of the expression level of the gene may preferably correspond to measurement of mRNA levels. Methods for measuring mRNA levels include, but are not limited to, reverse transcription polymerase chain reaction (RT-PCR), real-time reverse transcription polymerase chain reaction, RNase protection assay, northern blot, DNA chip, and the like.

Measurement of the protein activity level may use antibodies. The marker protein in the biological sample and the antibody specific thereto form a binding substance, that is, an antigen-antibody complex. The amount of the antigen-antibody complex as formed can be determined quantitatively using the intensity of the signal of the detection label. Such detection labels may be selected from the group consisting of enzymes, fluorescent materials, ligands, luminescent materials, microparticles, redox molecules and radioactive isotopes, and but may not be limited thereto. Analytical methods for measuring protein levels include Western blotting, ELISA, radioimmunoassay, radioimmunoprecipitation, and Ouchteroni immunodiffusion, rocket immunoelectrophoresis, tissue immunostaining, immunoprecipitation assays, complement fixation assays, FACS, protein chips, etc. The present invention is not limited to this method.

Further, the present disclosure provides a SETDB1 inhibitor for use in the treatment of cancer.

Terms not otherwise defined herein have meanings commonly used in the art to which the present disclosure pertains.

Hereinafter, the present disclosure will be described in detail using examples. However, the following examples are merely illustrative of the present disclosure, and the scope according to the present disclosure is not limited to the following examples.

### Materials and Methods

### Cell culture

Caco2, HCT116, SW480, SW620 human colorectal cancer cells were cultured in DMEM supplemented with 10% FBS and antibiotics (100 units/mL of penicillin, 100 µg/ml of streptomycin, and 0.25 µg/ml of Fungizone; Life Technologies Corp.) at 37°C in 5% CO₂. For retrovirus transduction, we cultured cells in above condition with 2 µg/ml of puromycin for three days after transduction and then cultured with 1µg/ml for the rest of the period. Three days after transduction, to activate PPARG and VDR, we supplemented Troglitazone (10 uM; T2573, Sigma Aldrich) and 1α,25-Dihydroxyvitamin D₃ (25 nM; D1530, Sigma Aldrich). We called this supplemented differentiation medium in the study.

### Gene expression data processing

We collected 17 independent gene expression data of normal colon mucosa performed by Affymetrix GeneChip Human Genome U133 Plus 2.0 (GEO-integrated normal dataset, n = 318; GSE71571, GSE23194, GSE37364, GSE41328, GSE33114, GSE23878, GSE20916, GSE15960, GSE9254, GSE4183, GSE8671, GSE11831, GSE32323, GSE9348, GSE4107, GSE10714, GSE18105) from the Gene Expression Omnibus (GEO) database. The data were normalized by robust multi-array average (RMA) method as implemented in the 'affy' package31 and batchcorrected with ComBat method as implemented in the 'sva' package32 in R. Also, the 11 Gene expression data of colorectal cancer samples performed by Affymetrix GeneChip Human Genome U133 Plus 2.0 (GEO-integrated colorectal cancer dataset, n = 2,166; GSE33113, GSE13067, GSE13294, GSE14333, GSE17536, GSE20916, GSE2109, GSE23878, GSE35896, GSE37892, GSE39582) from KFSYSCC dataset were downloaded and processed by same methods. In addition, we used the paired data of 98 adjacent normal colon mucosa samples and their tumor tissue samples performed by Affymetrix Human Genome U219 Array (GEO-paired dataset, n = 196; GSE44076).

For the Cancer Genome Atlas (TCGA) data, we used the transcriptome data by the format 'HTSeq-Fragments Per Kilobase of transcript per Million mapped reads upper quartile (FPKM-UQ)' downloaded from GDC Data Portal using 'TCGAbiolinks' package (TCGA dataset, n = 698). A Single-cell RNA-seq dataset was downloaded from the GEO database (Single-cell RNA-seq dataset, n = 432; GSE81861) and we selectively used the epithelial samples annotated by the previous study (Li, H. et al. 2017).

The data was performed by the SMARTer Ultra-Low RNA Kit for Illumina (Clontech, 634948) and sequenced using HiSeq 2000 platform (Illumina). Among the 1,591 cells from cancer cells and matched normal mucosa cells, they retained the 626 cells after QC (quality control). To further filter detectably expressed genes, we removed the genes that were measured to less than one tenth of the total samples.

### Gene sets

Tissue-specific gene sets were obtained from Human Protein Atlas (HPA), Genotype-Tissue Expression project (GTEx), and Functional Annotation of the Mammalian Genome (FANTOM) project and used. In detail, from HPA, we used the tissue specific gene lists which the study provides with the labels of "elevated genes" for each tissue. From GTEx, we selected the tissue-specific gene sets that have at least five-fold higher gene expression in a particular tissue as compared to all other tissues. Likewise, we defined the tissue-specific gene sets based on the same rule from the gene expression data of FANTOM5. The stem/progenitor associated gene sets were individually collected from the previous studies (Ben-Porath, I. et al. 2008; Wong, D. J. et al. 2008; Li, H. et al. 2017; Loh, K. M. et al. 2014; Kim, J. et al. 2010; Xie, R. et al. 2013).

### Differentially expressed gene (DEG) analysis

The DEG analysis was performed by using SAM (significance analysis of microarrays) method implemented in the 'siggenes' package in R. A delta value was set that false discovery rate (FDR) become zero in GEO-integrated (delta = 8.1) and GEO-paired datasets (delta = 15.9), and lower than 1.0e-06 in TCGA dataset (delta = 9.9), respectively. The common genes that showed higher expression in normal colon mucosa compared to CRC tissues across all three datasets were defined as normal-specific genes.

### Gene set enrichment analysis (GSEA)

GSEA (Gene set enrichment analysis) was performed using 'GSVA' package in R and GSEA code provided from Broad Institute. Gene set variation analysis (GSVA) implements a nonparametric, unsupervised method estimating variation of gene set enrichment over the samples without class labels. Each sample was independently assessed, and normal and cancer samples were compared across the datasets. The statistical significance is determined by P value of moderated t-statistics using 'limma' package in R. GSEA was performed to analyze RNA-seq data of three cell lines, evaluating the ES (enrichment scores) with distinct sample classes defined by experimental condition.

### Survival analysis

In the Kaplan-Meier survival analysis, the survival data were available for 633 CRC cancer patients from TCGA. Clinical information about overall survival and disease-free survival was downloaded from cBioPortal. We classified patients into two groups according to the median value of GSVA score of a colon specific gene set. Additionally, we further divided the groups with the third quantile of SETDB1 gene expression level. The analysis was performed using 'survival' package in R. Statistical significance was assessed using log-rank test.

### Principal component analysis of single-cell RNA sequencing data

To robustly estimate the state-specific gene expression profiles, we applied imputation algorithm MAGIC to recover the dropouts and performed principal component analysis (PCA) with the union of the state specific gene sets. In regard to colon-specific, intestinal stem cell-specific, and embryonic stem cell-specific gene sets, we independently performed PCA and used PC1 as a representative value.

### Pseudotime analysis

To reconstruct cellular trajectories of colorectal cancer, we used SLICER (Selective locally linear inference of cellular expression relationships) algorithm and Monocle2. SLICER performs nonlinear dimensionality reduction using locally linear embedding algorithm which calculates geodesic distances among the samples. The genes measured to more than one tenth of the total single-cell RNA sequencing samples were used to conduct dimensionality reduction in SLICER. For Monocle2, the differentially expressed genes across normal and cancer samples were determined with 0.01 cutoff of q value using the method implemented in 'monocle' package. Discriminative Dimensionality Reduction with Trees (DDRTree) method was used for performing Monocle2.

### Cell cycle analysis

Gene sets representing G₁/S and G₂/M phases of the cell cycle was defined in previous study (Tirosh, I. et al. 2016). As the previous study performed, we calculated the average expression of each gene set, and the cells of each phase were determined by the average expression value above 3.0 which results in the P value 1.5e-10 and 2.0e-16, respectively when assigned samples and remained samples were statistically compared.

### Transcription entropy analysis

The single-cell RNA-seq data were used as a read counts format. Median-normalization was performed using RaceID2 algorithm in R. The transcription entropy is defined as general formula for the information entropy where each probability indicates the number of transcripts of a single gene in a cell. The analysis was performed according to the previous study (Grun, D. et al. 2016).

### Network inference and identification of core transcription factors

To infer context specific gene regulatory networks (GRNs), we calculated mutual information of each dataset using ARACNE algorithm. In this case, we used a list of curated transcription factors from a previous study (Carro, M. S. et al. 2010) which are included in the list of transcription factors defined by FANTOM. We inferred GRNs from the GEO-integrated normal dataset and GEO-paired normal dataset and called them GEO-integrated normal GRN and GEO-paired normal GRN, respectively. To identify the master regulators of normal colon epithelial cells, we used Master Regulator Inference Algorithm (MARINA). We performed MARINA analysis with P value cutoff 1e-10. For GEO-integrated normal GRN and GEO-paired normal GRN, we identified the transcription factors whose inferred target genes were significantly enriched in colon-tissue specific genes and normal-specific genes, respectively. The common transcription factors which significantly include the colon-specific genes and normal-specific genes as their target genes were defined as the core TFs of normal colon epithelial cells. The transcriptional activity of the TFs was quantitatively measured using virtual inference of protein activity by enriched regulon analysis (VIPER) algorithm. We used a normalized enrichment scores (ES) calculated by single sample VIPER methods as a transcriptional activity.

### Identification of modulators using the inferred gene regulatory network

To identify the potential modulators, we established a set of criteria: a modulator 1) having negative regulatory interaction with the colon-specific genes, 2) having protein-protein interaction with the core TFs, and 3) inducing alteration of regulatory interaction between the core TFs and their target genes depending on the gene expression of a modulator. To measure the negative influence of potential modulators on the gene expression of colon-specific genes, we used VIPER algorithm. We reconstructed the regulatory network composed of proteins (GO: 0006464, cellular protein modification process, n = 2,477) using ARACNE algorithm in GEO-integrated cancer dataset. To define the putative modulators, we used the multiple sample version of VIPER with the colon-specific gene sets. We selected the proteins that have significant regulatory interaction with colon-specific genes with a P value below 0.01. To assess the protein-protein interaction, we used the STRING database. The interaction scores of each potential modulator was calculated by summing the value of 'combined interaction scores' of each modulator with the 5 core TFs. To examine the changes of regulatory interaction between each potential modulator and the core TFs, we assessed conditional mutual information (CMI) using Modulator Inference by Network Dynamics (MINDy) algorithm with P value cutoff threshold 1e-05. In detail, we used 300 samples among GEO-integrated cancer data with the highest gene expression of potential modulators, and 300 samples with the lowest gene expression of potential modulators. GEO-integrated normal GRN which has the largest number of samples among the datasets was used.

### Flow cytometry

*In vitro* cultured cells were fixed at 4% PFA and stained with KRT20 antibody (13063S, Cell Signaling) and FITC-anti rabbit IgG (554020, BD Pharmingen) according to the manufacture's instruction (Cat. No. 554714, BD Biosciences). To isolate RNA from intracellular immunofluorescent stained cells, we performed the experiments under RNase free condition with 1 : 100 RNasin Plus RNase Inhibitor (N2615, Promega) following intracellular sorting MARIS protocol. Flow cytometric analysis was conducted with a FACS Jazz (BD Biosciecnces) and analysis was performed with BD FACSDiva software.

### Immunofluorescence

For IF staining, cancer cells were fixed for 15 min in 4% (vol/vol) formaldehyde and blocked for 1 h in 2% BSA with 0.1% Triton-X100 of PBS, followed by incubation in primary antibody, KRT20 (13063S, Cell Signaling), and Ki-67 (11882S, Cell Signaling) at a dilution of 1 : 200 for staining buffer for overnight at 4°C. Samples were washed in PBS and incubated with the secondary antibody (ab150113, ab150080) at a dilution of 1 : 300 in staining buffer for 1 h. 4',6-diamidino-2-phenylindole (DAPI) was stained for 1 h before the confocal microscopic analysis. Imaging was performed a Nikon A1R confocal microscope (Nikon Instruments) and digital-zooming Nikon imaging software (NIS-element AR). Image data were analyzed by Nikon imaging software (NIS-element AR) and the number of Ki-67 positive cells were counted using MATLAB.

### shRNA, siRNA knockdown

Specific small interfering RNAs (siRNAs) against *EHMT2, HDAC2, KAT2A, KDM1A, PRMT1, SETDB1, SMYD2* and *SMYD3* were custom-synthesized (Bioneer) according to sequences provided from the previous studies. We used Scramble siRNA (Bioneer) as a control, and transfection was carried out using Lipofectamine RNAiMAX (Invitrogen). Retrovirus-mediated short hairpin RNA (shRNA) was purchased from Sigma-Aldrich. Detailed information on siRNA and shRNA experiments is given in Table 1 and Table 2.

**[Table 1]**

| **Name** | **Sequence (5' - 3')** | **SEQ ID NO** |
|---|---|---|
| shSETDB1#1 | CCGGGCTCAGATGATAACTTCTGTACTCGAGTACAGAAGTT ATCATCTGAGCTTTTTG | 1 |
| shSETDB1#2 | CCGGGCTCAGATGATAACTTCTGTACTCGAGTACAGAAGTT ATCATCTGAGCTTTTTG | 2 |
| shSETDB1#3 | CCGGAGTTAGAGACATGGGTAATACCTCGAGGTATTACCCAT GTCTCTAACTTTTTG | 3 |

**[Table 2]**

| **Name** | **Sequence (5' - 3')** | **SEQ ID NO** |
|---|---|---|
| EHMT2-S | CACACACCGACCAGAGA | 4 |
| EHMT2-AS | UCUCUGGUCGGUGUGUG | 5 |
| HDAC2-S | CGGGGCAACAACA | 6 |
| HDAC2-AS | UGUUGUUGCCCCG | 7 |
| KAT2A-S | GGAAAUGCAUCCUGCAGAU | 8 |
| KAT2A-AS | AUCUGCAGGAUGCAUUUCC | 9 |
| KDM1A-S | CACAAGGAAAGCAGAAGA | 10 |
| KDM1A-AS | UCUUCUGCUUUCCUUGUG | 11 |
| PRMT1-S | GGACAUGACAUCCAAAGAU | 12 |
| PRMT1-AS | AUCUUUGGAUGUCAUGUCC | 13 |
| SETDB1-S | CGGGGGCGCCAAAACAA | 14 |
| SETDB1-AS | UUGUUUUGGCGCCCCCG | 15 |
| SMYD2-S | GAUUUGAUUCAGAGUGACA | 16 |
| SMYD2-AS | UGUCACUCUGAAUCAAAUC | 17 |
| SMYD3-S | AGCCUGAUUGAAGAUUUGA | 18 |
| SMYD3-AS | UCAAAUCUUCAAUCAGGCU | 19 |

### Plasmid construction

For extrinsic expression of CDX2, ELF3, HNF4G, PPARG, and VDR, the full-length cDNAs of each gene were amplified by RT-PCR from human colon cDNA library (Clontech). Each cDNA was ligated into a pLentiM1.4 lentiviral vector and confirmed by sequencing.

### RNA isolation, quantitative RT-PCR and RNA sequencing

RNA was extracted using RNA-spin kit (INTRON) and cDNA was synthesized using Diaster RT kit (Solgent) and 2X Taq premix (Solgent) according to the manufacture's instruction. After reverse transcription, quantitative PCR was performed using the QuantStudio5 (Applied Biosystems) with SYBR Master Mix (GeNet Bio). PCR reactions were performed with three independent replicates and normalized using β-actin mRNA expression. All primer sequences used for RT-PCR are given in Table 3.

**[Table 3]**

| **Name** | **Sequence (5' - 3')** | **SEQ ID NO** |
|---|---|---|
| AQP8-F | GCC ATC AAT GAG AAG ACA AAG G | 20 |
| AQP8-R | CAC CTA ATG AGC AGT CCA ACA A | 21 |
| B-actin-F | AGA GCT ACG AGC TGC CTG AC | 22 |
| B-actin-R | AGC ACT GTG TTG GCG TAC AG | 23 |
| CA1-F | CCA AAC ATG ACA CCT CTC TGA A | 24 |
| CA1-R | AGC TCG GCA GAA TAT TTG ACT C | 25 |
| CD44-F | CTG CCG CTT TGC AGG TGT A | 26 |
| CD44-R | CAT TGT GGG CAA GGT GCT ATT | 27 |
| CDX2-F | TTC ACT ACA GTC GCT ACA TCA CC | 28 |
| CDX2-R | TCT GGG ACA CTT CTC AGA GGA C | 29 |
| CEACAM5-F | CAG ATC AGG GGA AAA TCT GAA C | 30 |
| CEACAM5-R | TCG TGA CTG TGG TCC TAT TGA G | 31 |
| EHMT2-F | TGC GTG CTG TTA TTC CTG TC | 32 |
| EHMT2-R | TGA TCT TCT CTG TGC GGA TG | 33 |
| ELF3-F | CAA AGA GTA CTG GGA CTG TCT CG | 34 |
| ELF3-R | CTC AGC TTC TCG TAG GTC ATG TT | 35 |
| FABP1-F | GCA GAG CCA GGA AAA CTT TG | 36 |
| FABP1-R | TCT CCC CTG TCA TTG TCT CC | 37 |
| FABP2-F | AAG CTT GCA GCT CAT GAC AA | 38 |
| FABP2-R | TCC ATT GTC TGT CCG TTT GA | 39 |
| GAPDH-F | TGA TGA CAT CAA GAA GGT GGT GAA G | 40 |
| GAPDH-R | TCC TTG GAG GCC ATG TGG GCC AT | 41 |
| HDAC2-F | GAG GTG GCT ACA CAA TCC GTA | 42 |
| HDAC2-R | ACA CCA GGT GCA TGA GGT AAC | 43 |
| HNF4G-F | GTT TCT TCA GAC GCA GCA TTC | 44 |
| HNF4G-R | AAC TTC AGC TTG TGC CAG TGT | 45 |
| KAT2A-F | GCT GAC CAC GTA TCC CAC TT | 46 |
| KAT2A-R | ATG CAT TTC CGC AGT AGC TT | 47 |
| KDM1A-F | TCAACT CTC TCC CTT AAG CAC TG | 48 |
| KDM1A-R | CAC AGC TAT CAC TTC ACA TCC TG | 49 |
| KRT20-F | ACG CCA GAA CAA CGA ATA CC | 50 |
| KRT20-R | ACG ACC TTG CCA TCC ACT AC | 51 |
| LGR5-F | CTC CCA GGT CTG GTG TGT TG | 52 |
| LGR5-R | GAG GTC TAG GTA GGA GGT GAA G | 53 |
| MYC-F | GGC TCC TGG CAA AAG GTC A | 54 |
| MYC-R | CTG CGT AGT TGT GCT GAT GT | 55 |
| PPARG-F | ATC TCT CCG TAA TGG AAG ACC A | 56 |
| PPARG-R | AGG CTC TTC ATG AGG CTT ATT G | 57 |
| PRMT1-F | ACC GCA ACT CCA TGT TTC A | 58 |
| PRMT1-R | CCA CCT TCC CCT TGA TGA T | 59 |
| SETDB1-F | CCG GCC TAC AGA AAT AAT TGA G | 60 |
| SETDB1-R | CAA GGT TCC TTT ATG CAG ATC C | 61 |
| SMYD2-F | GTG TAC CAC CAA GGA CAA GGA T | 62 |
| SMYD2-R | AGA TCT CCA GCA GCT CAC TAG G | 63 |
| SMYD3-F | AGA ACT GAA GGC ACA CTG GAA | 64 |
| SMYD3-R | TTC ATC ACT TGA ACC CCT CTG | 65 |
| VDR-F | AGA TGA CCC TTC TGT GAC CCT A | 66 |
| VDR-R | ATG GCA CTT GAC TTC AGC AGT A | 67 |

### Retrovirus production and transduction

For retrovirus-mediated gene transfer, HEK293T cells were transfected with each relevant lentiviral plasmid and packaging plasmids (pLP1, pLP2, and pLP/VSV-G, Invitrogen) using Lipofectamine (Invitrogen) according to the manufacturer's instructions. The medium was harvested after 48 h of transfection and filtered through a 0.22 µm cellulose acetate filter. Then, virus-containing medium was centrifuged at 25,000 r.p.m. for 90 min (Optima L90K, Beckman). Lentiviral packaged CDX2, ELF3, HNF4G, PPARG and VDR were concentrated in 2 ml of complete DMEM supplemented with 4 µg/ml polybrene and infected to HCT116 and SW480 cell lines. Lentiviral packaged shSETDB1 was infected to Caco2, HCT116, and W480 without concentration.

### Colony forming assay

For colony forming assay, 500 cells were seeded in 6-well plate and incubated for 14 d in medium supplemented with Troglitazone (10µM, T2573, Sigma Aldrich) and 1α,25-Dihydroxyvitamin D3 (25nM, D1530, Sigma Aldrich). Cells were fixed with 3.7% paraformaldehyde for 15 min, followed by staining with a 0.5% crystal violet solution for 1 h. After washing with distilled water and air drying, images were obtained.

### Cell growth assay

For assessing cell growth, transfected cells were seeded in 96-well plate (5 x 10³ cells/well) and imaged every 3h using IncuCyte ZOOM (Essen Bioscience). The confluence was analyzed by the software IncuCyte ZOOM 2016A.

### Microarray analysis

For Caco2, HCT116, SW480, and SW620, microarray analysis was performed on a single sample. Total RNA was extracted using RNA-spin kit (INTRON), and RNA purity and integrity were evaluated by ND-1000 Spectrophotometer (NanoDrop), Agilent 2100 Bioanalyzer (Agilent Technologies). The fragmented cRNA was hybridized to the Affymetrix GeneChip Human Genome U133 Plus 2.0 Gene Expression Array at 45°C for 16 h. Hybridized arrays were washed and stained on a GeneChip Fluidics Station 450 and scanned on a GCS3000 Scanner (Affymetrix). Signal values were computed using the Affymetrix^{®} GeneChip^{™} Command Console software. The data were normalized with robust multi-average (RMA) method.

### Bulk RNA sequencing analysis

Total RNA was extracted using RNA-spin kit (INTRON), and RNA quality was assessed by Agilent 2100 bioanalyzer using the RNA 6000 Nano Chip (Agilent Technologies). RNA quantification was performed using ND-2000 Spectrophotometer (Thermo Inc.), and 500 ng of RNA per sample was used for library construction. The library construction was performed using QuantSeq 3' mRNA-Seq Library Prep Kit (Lexogen Inc.) and the library was single-end sequenced on an Illumina NextSeq 500 (Illumina Inc.). The reads were aligned using Bowtie2 and the read counts estimated by Bedtools were further processed based on Quantile-Quantile normalization method using 'edgeR' package in R.

### Single-cell RNA sequencing analysis

For Caco2 cells stably expressed shRNA targeting SETDB1 and shScramble, we used 10X Genomics Chromium machine to capture single cells and cDNA preparation. 3000 cells were loaded on a Chromium Single Cell Instrument to generate single cell nanoliter-scale Gel Bead-In-EMulsions (GEMs). The barcoded cDNA was then amplified in PCR and the single-cell RNA sequencing libraries were prepared using version 1 Chromium Single Cell 3' Library, Gel beads and Multiplex kit (10X Genomics). The libraries were quantified using qPCR and qualified using the TapeStation D1000 ScreenTape (Agilent Technologies, Waldbronn, Germany). Indexed libraries were then sequenced using the HiSeq2500 platform (Illumina).

### Preprocessing and quality control of single-cell RNA sequencing data

We aligned raw FASTQ files to hg19 human genome assembly and aggregated two samples using the 10X Genomics Cell Ranger pipeline (https://support.10xgenomics.com/single-cellgene-expression/software/downloads/latest) with standard settings. Cells that had either the number of total counts under 20,000 or the number of expressed genes under 3,000 are precluded. After quality control, we obtained 453 cells of shSETDB1 samples and 654 cells of shScramble samples. To mitigate cell-specific biases, library size normalization was performed by 'scater' package, and consequent log-transformed normalized expression values were used following analysis.

### Example 1. Comparative analysis of normal colon tissue samples and colorectal cancer tissue samples

The GSEA (Gene Set Enrichment Analysis) was performed on approximately 600 normal colon tissue samples and approximately 3,000 colorectal cancer tissue samples extracted from the GEO database and the TCGA database. Colon-specific gene sets were extracted from Human Protein Atlas. Intestinal stem cell (ISC)-specific gene set and embryonic stem cell (ESC)-specific gene set were available from previous studies (Li et al. And Wong et al.). P-values were derived by performing a two-sample proportion test (*P<1e-10, **P<1e-30, ***P<1e-50). Further, local linear embedding (LLE) using SLICER was performed to infer cell trajectories from single cell RNA-seq data. Further, principal component analysis (PCA) was performed to classify expression patterns of each gene. Furthermore, the cell cycle of the whole cell population was analyzed and expressed as a scatter plot. The results are shown in FIG. 1 to FIG. 3.

As shown in FIG. 1, the most obvious feature of the cancer samples in all data sets was the expression reduction of the colon-specific gene set. Further, as shown in FIG. 2, through single cell analysis, it was identified that the cancer stem cells with high differentiation ability, and differentiated cancer cells without the differentiation ability are present in in the colon tissues. Further, as shown in FIG. 3, cells that underwent cell division were limited to cancer stem cells with high ability to differentiate.

### Example 2. Identification of clinical relevance of specific genetic expression patterns of normal colon cells

We identified whether the specific genetic expression pattern of normal colon cells has clinical relevance. Specifically, overall survival rate (n = 623, P-value = 0.011) and disease-free survival rate (n = 623, P-value = 0.0011) were analyzed using the Kaplan-Meier method. The GSVA score of the colon-specific gene set was dichotomized into a colon high or a colon low using the median as a cutoff. Further, the GSVA score of the colon-specific gene set based on the TNM staging system evaluated by the TCGA was represented by a box plot. The colon-specific gene set was provided from Human Protein Atlas as described in Example 1. The results are shown in FIGS. 4 to 5.

As shown in FIG. 4, we identified that the higher the expression level of the colon specific genes, which is higher in normal colon cells than the colorectal cancer cells, the higher the survival probability of the patients. Further, as shown in FIG. 5, we identified that expression levels of colon-specific genes were inversely related to the size of the patient's primary carcinoma.

### Example 3. Identification of core transcription factors of normal colon cells and expression level and activity analysis thereof

We performed identification of the core transcription factors of normal colon cells from two independent gene regulation networks deduced from public genomic data. Their expression levels and activities were compared and analyzed. Specifically, using a gene regulatory network (GRN) inferred from GEO (Gene Expression Omnibus)-integrated normal data set (GEO-integrated normal GRN) and GEO-paired normal data set (GEO-paired normal GRN), respectively, and by using MARINA, the core transcription factors regulating the colon-specific gene and the normal genes were derived (FIG. 6). Based on the GEO-integrated normal GRN, the core transcription factors, and their target genes were visualized (FIG. 7). The nodes were displayed in different colors based on the statistical significances of differences between normal and cancer cells tested with Student's t-test (unpaired, two-tailed).

Further, the Pearson correlation coefficient between each core transcription factor from the GEO-paired normal gene regulatory network and its regulon (a group of inferred target genes of transcription factor) is shown in a histogram (FIG. 8). Furthermore, we compared gene expression levels of core transcription factors in GEO-paired data sets. The activity of the core transcription factor inferred by the VIPER algorithm from the GEO-paired normal GRN and GEO-integrated normal GRN was analyzed (FIG. 9A to 9C). The results are shown in FIG. 6 to FIG. 9.

As shown in FIG. 6 and FIG. 7, from the results of the MARINA analysis, we have identified that CDX2, ELF3, HNF4G, PPARG, and VDR, the normal colon cell transcription factors with a P-value lower than 1e-10 are core transcription factors that determine specific gene expression patterns of differentiated normal colon cells. Further, as shown in FIG. 8 and FIG. 9, we have identified that expression levels and activity of the core transcription factors were significantly reduced in colorectal cancer cells compared to normal cells.

### Example 4. Detecting the gene that inhibits the activity of the transcription factor

As identified in Example 3, normal colon cell transcription factors play an important role in colorectal cancer cells. Thus, we explored potential regulators or chromatin regulators that inhibit the activity of core transcription factors that regulate specific gene expression patterns in normal colon cells. The results are shown in FIG. 10 and FIG. 11.

As shown in FIG. 10, we first performed a GO term enrichment analysis between the genes that have a negative correlation with the colon-related gene expression. Then, we selected 15 potential regulators. Further, as shown in FIG. 11, we analyzed STRING database and CMI scores that assessed the changes in the regulatory action between each potential regulator and core transcription factor. Based on these results, we selected eight potential regulators that showed high gene expression levels.

Then, we transfected 8 chromatin regulators in Caco2 cell as colon-derived colorectal adenocarcinoma cell, and epithelial cell with the siRNAs (siEHMT2, siHDAC2, siKAT2A, siKDM1A, siPRMT1, siSETDB1, siSMYD2, siSMYD3) listed in Table 2 above. We performed qRT-PCR (n = 3, mean±s.e.m.) to analyze the expression of differentiation markers and stem cell-related genes. As a result of the qRT-PCR analysis, SETDB1 showing the highest knockdown efficiency was selected as the final regulator. To identify long-term inhibitory effects of SETDB1 by retroviral-mediated shRNA interference, we designed an experiment for not only Caco2 cell but also HCT116 and SW480, a colorectal cancer cell line over-expressing all five core transcription factors. We used flow cytometry to identify the effect of SETDB1 deficiency on cell differentiation status. The shRNA represented by SEQ ID NO. 1 (shSETDB1 #1) as described in Table 1 was used. Further, immunofluorescence staining assyas and colony formation assays were performed to determine phenotypic characteristics of differentiated normal-like cells. The results are shown in FIGS. 12 to 17.

As shown in FIG. 12, when the expression of SETDB1 is suppressed, expression levels of differentiation markers such as KRT20, FABP1, FABP2, and CEACAM5 were greatly increased. At the same time, expression levels of stem cell-related genes such as LGR5, CD44 and MYC were greatly decreased. Further, as shown in FIG. 13, using real-time cell images, we have identified that the cell proliferation rate was markedly reduced upon SETDB1 knockdown. If the expression of SETDB1 is suppressed for a long time according to the experimental protocol shown in FIG. 14A, the cancer cell differentiation was induced by using flow cytometry (Fig. 14B). Using a confocal microscope image and a colony assay, we have identified at a single cell level that differentiated cancer cells expressing KRT20 differentiation markers are not actively proliferating (Fig. 15A to 15B). It was also identified that the growth of SETDB1 expression suppressed-cancer cells was inhibited at the population level (FIG. 16 and 17).

Using screening results from the RNA interference experiments, we have identified that the SETDB1 gene plays a major role in the inhibition of the activity of core transcription factors regulating the specific gene expression pattern of normal colon cells, and that the knockdown of SETDB1 in the colorectal cancer cell line expressing five core transcription factors induced the cells to differentiate from stem cell-like phenotypes to post-mitotic state.

### Example 5. Identification of various SETDB1 inhibitors converting cancer cells to normal cells

In order to more clearly identify that SETDB1 is the target regulating the division or differentiation of cancer cells, we have identified whether SETDB1 inhibitors effectively convert cancer cells to normal cells using two shRNAs (shSETDB1 # 2, SEQ ID NO. 2; shSETDB1 # 3, SEQ ID NO. 3 in Table 1) that target the SETDB1 gene and have different sequence therefrom, in addition to the shRNA represented by SEQ ID NO. 1. Specifically, in the same manner as described in Example 4 for each shRNA, we transfected the each shRNA into Caco2 cells and identified the effects thereof on division and differentiation of cancer cells. The results are shown in FIG. 18.

As shown in FIG. 18, both shRNAs (SEQ ID NO. 2 and SEQ ID NO. 3) inhibited SETDB1 expression (knock-down efficiency equal or above 50%). From the results of expression level of KRT20 using RT-PCR, which is a differentiation marker of the colon epidermal cell, we confirmed that the expression level of KRT20 was remarkably increased compared to the control group, and at the same time the expression level of MYC, which is a division and stem cell-related gene, was remarkably decreased.

From the results, we confirmed that the SETDB1 inhibitor is not limited to shRNAs targeting specific base sequences, and that cancer cells can be transformed into normal cells by inhibiting cancer cell division and promoting differentiation thereof in a cancer cell-specific manner using any method as long as the SETDB 1 gene expression is suppressed by the method.

### Example 6. Identification of gene expression in differentiated normal-like cells by SETDB1 inhibition

In order to identify whether SETDB1 depletion produced a global gene expression profile as in a normal differentiated colon cells, we performed single-cell RNA-seq and bulk RNA-seq analysis. For single-cell RNA-seq analysis, we obtained 453 cells and 654 cells, respectively, using Caco2 cells with shSETDB1 and shScramble introduced thereto. All in vitro samples, except the shScramble-Naïve sample, were cultured under differentiation conditions. The results are shown in FIGS 19 to 21.

As shown in FIG. 19 and FIG. 20, suppression of SETDB1 expression for one week again increased expression of colon-specific genes in the corresponding cells. Expression of the embryonic stem cell-specific gene and colon stem cell-specific genes decreased. Further, as shown in FIG. 21, when compared to patient data, the differentiated cancer cells were classified into normal cells, thereby indicating that the cancer cells successfully re-differentiated into normal cells. From the result, we identified via RNA-sequencing that cancer cells as differentiated successfully recovered the expression pattern of normal colon cells.

According to the results, we confirmed that SETDB 1 according to the present disclosure is a key protein that regulates the growth and differentiation ability of colorectal cancer cells, a type of solid cancer, and that the inhibition of the SETDB 1 may allow the malignity of the colorectal cancer cells to be markedly reduced, such that the colorectal cancer cells are converted to normal cells. Thus, the composition for regulating cancer cell division or differentiation comprising SETDB1 and a SETDB1 inhibitor may be applied to the development of new drugs such as new target therapy and anti-cancer drugs targeting the SETDB 1, and may be expected to have a large ripple effect on the related industries.

From the foregoing, it will be appreciated that various embodiments of the present disclosure have been described herein for purposes of illustration, and that various modifications may be made without departing from the scope and spirit of the present disclosure. Accordingly, the various embodiments disclosed herein are not intended to be limiting, with the true scope and spirit being indicated by the following claims.
Various preferred features and embodiments of the present invention will now be described with reference to the following numbered paragraphs (paras).
1. A composition for regulating cancer cell division or differentiation, the composition comprising SETDB1 (SET domain, bifurcated 1) or a SETDB1 inhibitor.
2. The composition of para 1, wherein the SETDB1 inhibitor is an expression inhibitor of SETDB 1 gene or an activity inhibitor of SETDB 1 protein.
3. The composition of para 2, wherein the expression inhibitor of the SETDB1 gene is selected from a group consisting of an antisense oligonucleotide, siRNA (small interfering RNA), shRNA (small hairpin RNA) and miRNA (microRNA) binding complementarily to mRNA of a gene for promoting expression of the SETDB1 or SETDB1 gene.
4. The composition of para 2, wherein the activity inhibitor of the SETDB1 protein is selected from a group consisting of an antibody, an antigen-binding fragment thereof, a compound, a peptide, a peptide mimetic, and an aptamer specifically binding to the SETDB1 protein.
5. The composition of para 1, wherein the SETDB1 inhibitor inhibits transcriptional activity of at least one protein selected from a group consisting of CDX2 (caudal type homeobox 2), ELF3 (E74 like ETS transcription factor 3), HNF4G (hepatocyte nuclear factor 4 gamma), PPARG (peroxisome proliferator-activated receptor gamma), and VDR (vitamin D receptor).
6. The composition of para 1, wherein the cancer cell is a cancer stem-like cell.
7. A pharmaceutical composition for prevention or treatment of cancer, the composition comprising a SETDB1 inhibitor.
8. The pharmaceutical composition of para 7, wherein the cancer is at least one selected from a group consisting of colon cancer, rectal cancer, liver cancer, stomach cancer, lung cancer, breast cancer, and pancreatic cancer.
9. The pharmaceutical composition of para 7, wherein the pharmaceutical composition is used for differentiation treatment of cancer cells or cancer stem-like cells.
10. The pharmaceutical composition of para 7, wherein the pharmaceutical composition is used as a combination drug with an anti-cancer agent or a radiotherapeutic agent.
11. An *in vitro* method for inducing differentiation of cancer stem-like cells into normal cells or normal-like cells, the method comprising applying SETDB1 or a SETDB1 inhibitor to the cancer stem-like cells.
12. A method for screening an agent for preventing or treating cancer, the method comprising:
   (a) applying a candidate substance to an isolated cancer cell expressing SETDB1;
   (b) measuring a SETDB1 expression or activity level in the isolated cancer cell to which the candidate substance is applied; and
   (c) selecting the candidate substance as an agent for preventing or treating cancer when the SETDB 1 expression or activity level measured in the step (b) is lower than a SETDB 1 expression or activity level in an isolated cancer cell to which the candidate substance is not applied.
13. A SETDB 1 inhibitor for use in the treatment of cancer.

## Claims

1. A SETDB1(SET domain, bifurcated 1) inhibitor for use in the treatment of colon cancer, wherein the SETDB 1 inhibitor induces differentiation of the colon cancer cells.

2. The SETDB1 inhibitor for use according to claim 1, wherein the SETDB1 inhibitor increases the activity of at least one protein selected from the group consisting of CDX2(caudal type homeobox 2), ELF3 (E74 like ETS transcription factor 3), HNF4G(hepatocyte nuclear factor 4 gamma), PPARG (peroxisome proliferator-activated receptor gamma) and VDR(vitamin D receptor).

3. The SETDB1 inhibitor for use according to claim 1 or claim 2, wherein the colon cancer cells are colon cancer stem-like cells.

4. The SETDB1 inhibitor for use according to any preceding claim, wherein the SETDB 1 inhibitor increases expression levels of one or more differentiation markers selected from the group consisting of KRT20, FABP1, FABP2 and CEACAM5.

5. The SETDB1 inhibitor for use according to any preceding claim, wherein the SETDB 1 inhibitor reduces expression levels of one or more stem cell-related genes selected from the group consisting of LGR5, CD44 and MYC.

6. The SETDB 1 inhibitor for use according to any preceding claim, wherein the SETDB1 inhibitor is selected from the group consisting of an antisense oligonucleotide, siRNA(small interfering RNA), shRNA(small hairpin RNA), miRNA(microRNA), an antibody or an antigen-binding fragment thereof, a compound, a peptide, a peptide mimetic, and an aptamer.

7. The SETDB1 inhibitor for use according to any preceding claim, wherein the SETDB1 inhibitor is selected from the group consisting of shRNAs represented by sequences of SEQ ID Nos 1 to 3 and siRNAs represented by sequences of SEQ ID Nos 14 to 15.

8. The SETDB1 inhibitor for use according to any preceding claim, wherein the SETDB 1 inhibitor is provided in a pharmaceutical composition.

9. The SETDB1 inhibitor for use according to any preceding claim, wherein the SETDB1 inhibitor is used as a combination drug with an anti-cancer agent or a radiotherapeutic agent.
